Europäisches Patentamt

European Patent Office

Office européen des brevets

(11) Veröffentlichungsnummer: **0 382 848**
**A1**

(12) **EUROPÄISCHE PATENTANMELDUNG**
**veröffentlicht nach Art. 158 Abs. 3**
**EPÜ**

(21) Anmeldenummer: 89902309.7

(22) Anmeldetag: 25.01.89

(86) Internationale Anmeldenummer:
**PCT/SU89/00021**

(87) Internationale Veröffentlichungsnummer:
**WO 89/07134 (10.08.89 89/18)**

(51) Int. Cl.⁵: **C12N 5/00, A61K 39/395**

(30) Priorität: 03.02.88 SU 4395435

(43) Veröffentlichungstag der Anmeldung:
**22.08.90 Patentblatt 90/34**

(84) Benannte Vertragsstaaten:
**BE DE FR GB SE**

(71) Anmelder: **INSTITUT IMMUNOLOGII**
**Kashirskoe shosse, 24-2**
**Moscow, 115478(SU)**

(72) Erfinder: **ZLOBINA, Elena Nikolaevna**
**ul. Malygina, 9-187**
**Moscow, 129345(SU)**

(74) Vertreter: **Kolb, Helga, Dr. Dipl.-Chem. et al**
**Hoffmann, Eitle & Partner Patentanwälte**
**Arabellastrasse 4**
**D-8000 München 81(DE)**

(54) **STAMM VON C10G7-HYBRIDOMEN, DER MONOKLONALE ANTIKÖRPER PRODUZIERT, DIE GEGEN DIE FAMILIE VON BETA-ZELLEN-ANTIGENEN DES PANKREAS MIT EINEM MOLEKULARGEWICHT VON 64-96 KD GERICHTET SIND.**

(57) A strain of hybridome C10G7-producer of monoclone anitbodies to the family of beta-cell antigens of
the pancreas having a molecular mass of 64-69kD is
deposited on 28.01.88 with the USSR Research Institute for Antibiotics (VNIIA) under No.
VSKK(P)251D.

HYBRIDOMSTAMM C10G7 - PRODUZENT DER MONOKLONALEN ANTIKÖRPER GEGENÜBER DIE FAMILIE DER ANTIGENE DER PANKREAS-β-ZELLEN VON 64 BIS 69 kD MOLEKULARMASSE

## Gebiet der Technik

Die Erfindung bezieht sich auf ein Gebiet der Medizin und betrifft insbesondere einen neuen Hybridomstamm C 10 G7 - den Produzenten der monoklonalen Antikörper gegenüber die Familie der Antigene der Pankreas-β-Zellen von 64 bis 69 kD Molekularmasse.

## Zugrundeliegender Stand der Technik

In der klinischen und experimentellen Immunologie sind die monoklonalen Antikörper für die Identifizierung der mit einem konkreten pathologischen Prozeß assoziierten antigenen Markierungsmittel von verschiedenen Organen und Geweben von entscheidender Bedeutung.

Im Bestand der Autoantikörper, die im Blutserum der Diabeteskranken vom Typ I enthalten sind, sind die Antikörper, die das Antigen der Pankreasinselzellen markieren und eine Molekularmasse von 64 kD haben, vom größtem Interesse. Die Expression dieses Antigens ist mit der Entwicklung des Diabetes mellitus vom Typ I streng assoziiert. Deswegen hat in der Untersuchung und Diagnostik des Diabetes mellitus die Erhaltung der monoklonalen Antikörper, die das genannte Antigen markieren, und der Beweis mit Hilfe der monoklonalen Antikörper der Expression dieses Antigens in der Population der Inselzellen auf den β-Zellen des Pankreas eine große Bedeutung.

Zur Zeit sind die Hybridomstämme 2G3, 3G3, BI bekannt, die die Antikörper gegenüber die Antigene der Inselzellen produzieren, die eine Molekularmasse von 21, 27 und 66 kD haben (H. Vissing, G. Papadopoulos & Lernmark, Monoclonal Antibodies aganist Pancreatic Islet-Cell-Cerface Antigen Selected by Flow Cytofluorometry, Scand. J. Immunol., 1986, 23, 425--433). Keiner von diesen Stämmen weist jedoch ein für die β-Zellen streng spezifischen Antigen auf, weil die monoklonalen Antikörper mit den Epithelzellen einer Reihe von anderen Organen reagieren, d. h. sie besitzen eine mehrzählige Organenreaktivität. Es ist auch der Stamm

- 2 -

HISL-19 (S. Srikanta, K. Krisch and G. S. Eisenbarth, Islet Cell Proteins Defined by Monoclonal Iselt Cell Antibody HISL-19, Diabetes 1986, vol, 35, pp. 300 - 304), von 120, 69 67 und 56 kD Molekularmasse bekannt, der eine ganze Gruppe verschiedener Proteine der Inselzellen aufweist. Außer den Inselzellen reagieren jedoch diese monoklonalen Antikörper mit den Zellen anderer Endokrinorgane (der Schilddrüse, des vorderen Hypophyselappens u. a.). Außerdem wurde im Bereich der Population der Pankreasinselzellen keine enge Assoziation der monoklonalen Antikörper mit den B-Zellen gezeigt.

Dadurch ist es möglich, mit Hilfe dieser Antikörper ein für die Insulin-produzierenden Zellen spezifisches antigenes Markierungsmittel zu isolieren.

Es ist auch der Stamm 3A4, der monoklonale Antikörper gleichzeitig gegenüber zwei Polypeptide auf den Pankreasinselzellen produziert, mit der Molekularmasse von 64 und 28 kD bekannt (J. Hari and associates, Immuno-cheminal characteristics of monclonal ICSA, Diabetes, 1986, vol. 35, p. 517 - 522; Folia endocrinol., Japan, 1985, 61, 56 - 63).

Die monoklonalen Antikörper, die vom Stamm 3A4 produziert werden, gestatten jedoch nicht, das reine Antigen von 64 kD Molekularmasse zu isolieren, weil sie gleichzeitig mit zwei Determinanten der Pankreas-Inselzellen - mit der oberflächlichen von 64 kD Molekularmasse sowie mit der intrazellulären von 28 kD Molekularmasse gebunden werden, d. h. sie besitzen keine Spezifität in bezug auf das pathogenetische antigene Markierungsmittel des Diabetes mellitus vom Typ I - das Antigen von 64 kD Molekularmasse.

Deswegen können sie zur Immunodiagnostik des Diabetes mellitus nicht verwendet werden.

Außerdem ist die aus den Lymphozyten des Kranken im prädiabetischen Zustand erhaltene Linie der Zellen MC4E4 (C. R. Acad.Sc. Paris, 1985, t. 301, Serie III, n°13, 611 615) bekannt, die mit dem Virus von Epstein-Barr transformiert wurden. Ungeachtet dessen, daß die von ihnen produzierten monoklonalen Antikörper Antigene von 64 kD und 56 kD Molekularmasse aufweisen, besaßen sie keine strenge

- 3 -

Spezifität in bezug auf die ß-Zellen des Pankreas und wirkten mit anderen Endokrinzellen sowie mit Fibroblasten und der Linie des Insulinoms der Ratte RINm5F zusammen.

Zur Zeit sind keine Stämme der kultivierbaren Hybridzellen - Produzente der monoklonalen Antikörper bekannt, die gegenüber das Antigen der Pankreas-ß-Zellen streng spezifisch wären.

Offenbarung der Erfindung

Die Erfindung liegt die Aufgabe zugrunde, einen neuen Stamm des Hybridoms zu entwickeln, der den monoklonalen Antikörper produziert, in bezug auf das Antigen der Pankreas-ß-Zellen spezifisch ist, in der Medizin in der Immunodiagnostik und Prophylaxe des Diabetes mellitus sowie als Matrix für die gentechnische Synthese Verwendung findet.

Die Aufgabe würde dadurch gelöst, daß ein neuer Hybridomstamm C10 G7 - der Produzent der monoklonalen Antikörper gegenüber die Familie der Antigene der Pankreas-ß-Zellen vorgeschlagen wird, der eine Molekularmasse von 64 bis 69 kD hat, am 2Ü.01.88 im Allunions-Forschungsinstitut für Antibiotika (ЛНИИА) hinterlegt wurde und unter der Nummer ВСКМ (russisch abgekürzt) (P) 25ID registriert wurde.

Der erfindungsgemäße Stamm sekretiert den monoklonalen Antikörper, der. die xenogene Familie der Antigene von 64 bis 69 kD Molekularmasse aufweist, für die Pankreas-ß-Zellen spezifisch ist, in der Entwicklung der autoimmunen Komponente bei der Erkrankung am Insulin-abhängigen Diabetes mellitus von der entscheidenden Bedeutung ist. Mit Hilfe des genannten Antikörpers gelingt es, die xenogene Familie der Antigene von 64 bis 69 kD Molekularmasse zu isolieren, die für die Insulin-produzierenden Pankreaszellen des Menschen und der Ratte spezifisch ist, und diese in der Immunodiagnostik des Diabetes mellitus vom Typ I und zur gentechnischen Synthese des Proteins - des Markierungsmittels des Diabetes mellitus vom Typ I einzusetzen, um ein Immunodiagnostikum der neuen Generation zu entwickeln.

Monoklonale Antikörper werden auch zur Entwicklung eines adäquaten Modells des experimentellen autoimmunen Diabetes mellitus vom Typ I ausgenutzt, um neue Schemen der Therapie durchzuarbeiten und die Pathogenese dieser Erkrankung beim Menschen an den Tag zu legen.

## Beste Ausführungsform der Erfindung

Der erfindungsgemäße Hybridomstamm, der bedingt CIOG7 genannt wurde, produziert monoklonale Antikörper gegenüber die Familie der Antigene von 64 bis 69 kD Molekularmasse, die die Pankreas-3-Zellen markiert.

Die monoklonalen Antikörper, die vom erwähnten Hybridom CIOG7 produziert werden, bedingt ICA-I genannt werden, reagieren mit dem azinären Gewebe des Pankreas, mit den Zellen der Leber, des Bluts, des Zwölffingerdarms, des Thymus, der Milz, der Hypophyse, der Schild- und Speicheldrüse nicht.

Die Antikörper ICA-I markieren ausschließlich die Zonen der insulinhaltigen ß-Zellen der Pankreasinsel.

Der erfindungsgemäße Stamm wird folgenderweise erhalten.

Die Mäuse der Gattung BALB/c werden mit $2 \times 10^7$ Inselzellen intraperitoneal immunisiert, die bei der Verdauung des Pankreas der neugeborenen Ratten der Wistar-Gattung mit Kollagenase-Trypsin erhalten wurden. Nach 24 Tagen wird die Immunisation ähnlicherweise wiederholt und nach 14 Tagen werden dieselben Zellen intravenös eingeführt. Nach noch 4 Tagen werden die Milzzellen der Immunmäuse mit den Zellen des Mäusenmyeloms P3-X6X-Ag8.653 mit Hilfe des 50%igen Polyäthylenglykol (von 1500 Molekularmasse) vereinigt. Das Verhältnis der Milzzellen und Myelomzellen beträgt 5:1. Nach der Hybridisation warden die Zellen in Plättchen mit 96 Aushöhlungen zu je $10^5$ Zellen pro 1 Aushöhlung ausgesät, in die vorläufig peritoneale Makrophagen der Maus BALB/c zu je $10^4$ Zellen pro 1 Aushöhlung ausgesät wurden. Die Selektion des Hybridoms wird im durch Dulbecco modifizierten Minimalmedium nach Ieagle durchgeführt, das 10 % embryonales Kalbsblutserum, $10^{-4}$ M Hypoxanthin, $4 \times 10^{-7}$ M Aminopterin und $1,6 \times 10^{-5}$ Thymidin enthält. Das Hybridom wird zweimal in einer Menge von 1 Zelle pro 1 Aushöhlung kloniert. Der Klonenanteil, der die Produktion der Antikörper von der aufgegebenen Spezifität aufrechterhält, ist mindestens 90 % bei beiden Klonieren.

Der erfindungsgemäße Stamm wird durch folgende Merkmale gekennzeichnet.

## Kulturmerkmale

Das Medium fürs Kultivieren: das Minimalmedium nach Ieagle, modifiziert durch Dubecco, welches 10 % embryonales Kalbsblutserum, 2 mM L-Glutamin, 100 Einh./ml Gentamyzin und $5 \times 10^{-5}$ M 2-Merkaptoäthanol enthält. Die optimale Zellenkonzentration beim Kultivieren beträgt $5 \times 10^5$ Zellen/ml. Die Impfdosis beträgt $10^5$ Zellen/ml. Die Subkultivierungszeit beträgt 3 bis 4 Tage. Das Hybridom lebt und vermehrt sich gut unter Standardbedingungen für die Züchtung des Ausgangsmyeloms. Die Zellen halten das 7 Tage langes Kultivieren ohne Ersetzen des Nährmediums beim Ausgangssäen von $50 \times 10^5$ Zellen/ml unter einer allmählichen Herabsetzung der Lebensfähigkeit bis 20--30 % nach der Periode des Maximalanwachsens aus. Die Zeit der Kulturverdoppelung, die durch die direkte Berechnung der Zellenmenge in 1 ml Suspension bestimmt wurde, verändert sich von 24 bis 78 Stunden je nach den Bedingungen der Züchtung.

## Morphologie der Zellen

Die Zellen sind dem Ausmaß nach gleichartig, von der abgerundeten Form. Die meisten Zellen haben einen abgerundeten Kern. Die Kerne sind groß, vorzugsweise im Zentrum der Zellen angeordnet und etwas zur Peripherie verschoben. Die Kerne besetzen den größten Teil des Zellenvolumens. Das Zytoplasma ist in Form von einem engen Rand rings um den Kern herum angeordnet. Bei der Färbung nach Romanowski-Giemsa hat der Kern eine rötlich-violette Färbung, das Zytoplasma - eine blaue Färbung. Es werden keine Einschlüsse im Zytoplasma beobachtet.

## Die Züchtung in vivo.

Beim Passieren des Hybridoms an Mäusen der Gattung BALB/c beträgt die Zellendosis bei der Impfung $5 \times 10^6$ Zellen.

- 6 -

Die Zeit der Aszitesbildung beträgt 8 bis 12 Tage.

Die Charakteristik der Zellenspezifität des Zielproduktes.

Die durch den erfindungsgemäßen Stamm produzierbaren monoklonalen Antikörper reagieren spezifisch mit der Familie der oberflächen Antigene der Pankreas-ß-Zellen von 64 bis 69 kD Molekularmasse und reagieren nicht mit den Membranen der Blutzellen, Thymozyten, Splenozyten, den Zellen der Schild-, Speicheldrüse und der Hypophyse, mit azinären Zellen des Pankreas, mit den Leberzellen.

Die immunchemische Charakteristik der monoklonalen Antikörper.

Die monoklonalen Antikörper ICA-I gehören zur Klasse der Immunoglobuline G, Isotyp Ig G 2b.

Kontamination. Die Kontamination der Bakterien, Pilzen und Mykoplasmen wurde nicht nachgewiesen.

Die Konservierung der Zellen.

Das Hybridom wird im Kulturmedium kryokonserviert, das 50 % embryonales Kalbsblutserum und 10 % Dimethylsuloxid enthält. Das Regime der Gefrierkonservierung: I Grad pro 1 min bis -70°C, weiterhin werden die Zellen in den flüssigen Stickstoff übertragen. Das Hybridom wird beim Übertragen der Ampulle mit den Zellen aus dem flüssigen Stickstoff ins Wasserbad bei 37°C aufgetaut.

Die Lebensfähigkeit der Zellen nach der Aufbewahrung im flüssigen Stickstoff beträgt innerhalb von einem Jahr mindestens 70 %, beurteilt nach dem Einschluß des Farbstoffes Eosin und Trypanblau.

Zum besseren Verständnis der vorliegenden Erfindung werden folgende Beispiele angeführt, die den erfindungsgemäßen Stamm kennzeichnen.

Beispiel 1

Das Hybridom wird bei einer Dichte von $5 \times 10^5$ Zellen/ml in eine Plasteflasche mit dem durch Dulbecco modifizierten Minimalmedium nach Ieagle geimpft, das 10 % embryonales Kalbsblutserum, 2mM L-Glutamin, 100 Einh./ml Gentamyzin und $5 \times 10^{-5}$ M 2-Merkaptoäthanol enthält. Die Zellen werden innerhalb von 3 bis 4 Tagen bei einer Temperatur von 37°C

- 7 -

in der Atmosphäre mit 5 % $CO_2$ gezüchtet. Nachher wird die Kulturflüssigkeit gesammelt, bei einer Geschwindigkeit von 3000 U/min innerhalb von 20 min zentrifugiert und das Supernatant wird als Antikörperquelle ausgenutzt.

Die Pankreas von 30 neugeborenen Ratten der Wistar-Gattung werden mit Kollagenase-Trypsin, dann mit Trypsin-Äthylenglykol-N,N,N',N'-tetraesseigsäure-bis-(3-amino-äthyl)ester - Desoxyribonuklease behandelt. Ähnlicherweise wird die Kultur der Inselzellen der menschlichen Embryonen (die 12 bis 24 Wochen lange Schwangerschaft, spontaner Abort) erhalten.

Die Zellen werden in dem durch Dulbecco modifizierten Ieagle Medium mit 10 % embryonales Kalbsblutserum untergebracht bei einer Temperatur von 37°C innerhalb von 18 Stunden in der Atmosphäre mit 5 % $CO_2$ auf den Plasten-Petrischalen gezüchtet. Dann werden die Zellen von den Petrischalen abgenommen und mit einer Lösung abgewaschen, die 0,15 M NaCl, 0,01 M Natriumphosphat, bei einem pH-Wert von 7,2 enthält. Die Zellen werden gezählt. $10^6$ Zellen werden innerhalb von 30 min bei einer Temperatur von 37°C im angegebenen Medium inkubiert, aus welchem nachher eine Probe auf die Insulinproduktion entnommen wird. Das Niveau der basalen Insulinsekretion pro $10^6$ Inselzellen beträgt 560±65 µUml, $0,5x10^6$ Inselzellen werden innerhalb von 30 min bei einer Temperatur von 4°C in 50 µl Supernatant inkubiert, das monoklonale Antikörper enthält. Die Zellen werden zweimalig mit einer Lösung abgewaschen, die 0,15 M Natriumchlorid, 0,01 M Natriumphosphart und 0,01 % Natriumazid bei einem pH-Wert von 7,2 abgewaschen, bei einer Geschwindigkeit von 1000 U/min innerhalb von 10 min zentrifugiert und in 50 µl Antikörper gegenüber IgG der Maus suspendiert, die mit Fluoreszeinisothiozynat konjugiert sind. Nach 30 min Inkubation bei einer Temperatur von 4°C werden die Zellen, wie oben gezeigt, abgewaschen, in 100 µl 1%iges Paraformaldehyd suspendiert. Die Fluoreszenz wird mit Hilfe eines Durchflußzytometers registriert. Der Anteil der leuchtenden Zellen wird bei der Berechnung von 20 000 Inselnzellen bestimmt.

- 8 -

Als negative Kontrolle wird die Messung der Reaktivität der Inselzellen mit monoklonalen Antikörpern von demselben Isotyp (IgG 2b) von der anderen Spezifität (gegenüber das allgemein leukozytäre Antigen) ausgenutzt.

Die als negative Kontrolle genommenen monoklonalen Antikörper IgG2b des Isotyps gegenüber das allgemein leukozytäre Antigen reagieren mit den Inselzellen nicht.

Beim 20-fachen Screening auf den innerhalb von 18 Stunden kultivierten Inselzellen der Ratte wurde die Bindung von $76\pm6,1$ % der Zellen mit den monoklonalen Antikörpern ICA-I nachgewiesen, die vom erfindungsgemäßen Hybridomstamm produziert werden. Beim 3fachen Screening der innerhalb von 18 Stunden kultivierten Inselzellen des Menschen wurde die Bindung von $54\pm5$ % der Zellen mit den monoklonalen Antikörpern ICA-I festgestellt.

Als Kontrolle wurde die Reaktion der genannten monoklonalen Antikörper mit anderen Endokrinzellen durchgeführt.

Es wurde nachgewiesen, daß die monoklonalen Antikörper ICA-I mit anderen Endokrinzellen (der Speichel-, der Schilddrüse, der Hypophyse) sowie mit den Zellen der nichtendokrinen Herkunft - Thymozyten, Splenozyten, den Zellen des peripheren Blutes nicht zusammenwirken. Diese Antikörper reagieren auch mit der Linie des Ratteninsulinoms RINm5F nicht.

Beispiel 2

Das Pankreasfragment des Menschen oder der Ratte der Wistargattung von $0,5\times0,5$ cm$^2$ Größe wird im flüssigen Stickstoff eingefroren und nachher in der abgekühlten Kammer des Kryostats bei einer Temperatur von -20°C untergebracht, und es werden Kryoschnitte von 6 bis 8 um Dicke bereitgestellt. Jeder Schnitt wird auf ein Glas mit 50 γ Poly-I-lysin aufgetragen und innerhalb von 1 sec durch die Erwärmung bis 36°C fixiert. Die Schnitte werden im wasserfreien Azeton innerhalb von 7 bis 10 min fixiert. Danach werden sie in tris-NaCl-Lösung übertragen und innerhalb von 6 min inkubiert. Die Schnitte werden in einer feuchten Kammer untergebracht, darauf werden zu je 50 μl Supernatant des Hybridoms CIOG7 aufgetragen und diese wer-

-9 -

den innerhalb von 60 min bei Zimmertemperatur inkubiert. Dann werden siedreimal durch das Eintauchen in ein Gefäß mit tris-NaCl für 5 min abgewaschen. Nachher werden auf die Schnitte zu je 50 µl mit Peroxydase konjugierte Antikörper gegenüber IgG der Maus aufgetragen und innerhalb von 60 min bei Zimmertemperatur inkubiert. Es wird das Abwaschen nach dem oben angezeigten Verfahren wiederholt. Es wird unter der Sichtskontrolle die Nachfärbung mit Diaminobenzidin durchgeführt. Das Produkt der Immunperoxydasefärbung der Inselzellen mit den monoklonalen Antikörpern wird mit Hilfe des Lichtmikroskops berücksichtigt. Beim 15-fachen Screening auf den Pankreasschnitten der Ratten der Wistar-Gattung und beim 5-fachen Screening auf den Pankreasschnitten des Menschen wurde die Färbung der Inselzellen in der Zone der Anordnung der B-Zellen vom Membrancharakter festgestellt. Auf den Schnitten anderer Endokrindrüsen sowie des Darms, der Leber, der Milz, des Thymus wurde keine Reaktivität mit den gegebenen monoklonalen Antikörpern festgestellt.

Beispiel 3

Um die Spezifität der monoklonalen Antikörper ICA-I gegenüber die Pankreas-B-Zellen zu beweisen, wurde ein Doppelfärbung der Inselzellen mit den monoklonalen Antikörpern ICA-I und monoklonalen Antikörpern gegenüber Insulin durchgeführt. Die Zellenkultur wurde ähnlich mit dem Beispiel 1 erhalten. Die Immunofluoreszenzreaktion wird aus 4 nacheinanderfolgenden Etappen durchgeführt.

I. Das Inkubieren mit den monoklonalen Antikörpern gegenüber Insulin (der Mäuseherkunft).

2. Das Inkubieren mit dem mit Fluoreszein markierten Antiblutserum gegenüber IgG der Maus.

3. Das Inkubieren mit dem (vorläufig biotinilierten) monoklonalen Antikörpern ICA-I.

4. Das Inkubieren mit Streptavidin-Phykoerythrin.

Die Termine, die Inkubations- und Abwaschenbedingungen sind mit denen im Beispiel 1 beschriebenen ähnlich.

Es wird die Messung der Fluoreszenz am Durchflußzytometer mit 2 Engbandfiltern durchgeführt, die gestattet, die rote (bei 560-576 nm) und grüne (bei 510-530 nm) Fluoreszenz getrennt zu berücksichtigen. Im Ergebnis wurde nachgewiesen, daß 50 % der Zellen mit den monoklonalen Antikörpern ICA-I und 70 % mit den Antiinsulinantikörpern zusammenwirken; alle Zellen, die die Familie der Antigene IC ICA-I expressieren, reagieren positiv mit den Antiinsulinantikörpern. Die monoklonalen Antikörper ICA-I wirken also spezifisch mit den Pankreas-β-Zellen zusammen.

Beispiel 4.

Um die Molekularmasse der Familie der Antigene zu klären, die mit den monoklonalen Antikörpern ICA-I zusammenwirken, wird deren Ausscheidung aus Lysaten der Inselzellen der Ratte nach der Methode der affinen Chromatographie ausgenutzt.

Zur Bereitstellung von 1m 1 Lysat in nichtionischen Detergens NP-40 werden $20x16^6$ kultivierte Inselzellen ausgenutzt, die ähnlich mit dem Beispiel 1 erhalten werden. Die monoklonalen Antikörper ICA-I werden in einer Kolonne mit der Protein-A-Sefarose gereinigt.

Pro 1 ml Immunosorbens (Protein A-gereinigte monoklonale Antikörper ICA-I, die mit Brom-Zyansefarose konjugiert wurden) werden 20 ml Lysat genommen. Nach dem Passieren durch diese Kolonne wird das Protein eluiert, das nach der Methode der SDS-Elektrophorese und der zweidimensionalen SDS-Elektrophorese analysiert wird.

Im Ergebnis wurden 3 eng gebundene Proteine mit einer Molekularmasse von 69,67 und 64 kD bestimmt, die einen isoelektrischen Punkt (pI 6,5) haben, was den Anlaß gibt, diese Proteine zur einheitlichen Familie der Antigene p64-69 zuzurechnen.

Industrielle Verwendbarkeit

Der neue Hybridomstamm CICG7 - der Produzent der monoklonalen Antikörper gegenüber die Familie der Antigene der Pankreas-β-Zellen findet Verwendung in der Medizin zur Immunodiagnostik des Diabetes mellitus vom Typ I, kann zur Untersuchung der Pathogenese des Insulin-abhängigen Diabetes mellitus ausgenutzt werden. Die monoklonalen Antikörper,

- 11 -

die von erfindungsgemäßen Stamm produziert werden, können zur gentechnischen Synthese des Proteins - des Markierungsmittels des Diabetes mellitus vom Typ I zwecks der Entwicklung eines Immunodiagnostikums der neuen Generation ausgenutzt werden.

Die isolierte Familie der Antigene fand Verwendung in der Immunodiagnostik des Diabetes mellitus vom Typ I und kann auch zur Erarbeitung der Maßnahmen und der Behandlungsmethoden verwendet werden, die der Entwicklung des Diabetes mellitus vorbeugen.

- 12 -

PATENTANSPRUCH:

Hybridomstamm CIOG7 - Produzent der monoklonalen Antikörper gegenüber die Familie der Antigene der Pankreas-ß-Zellen von 64 bis 69 kD Molekularmasse, das am 26,01,88 im Allunions-Forschungsinstitut für Antibiotika (WNIIA) hinterlegt wurde und unter der Nummer BCKK (P) 251D registriert wurde.

# INTERNATIONAL SEARCH REPORT

International Application No PCT/Su 89/00021

## I. CLASSIFICATION OF SUBJECT MATTER (if several classification symbols apply, indicate all) [6]

According to International Patent Classification (IPC) or to both National Classification and IPC

$IPC^4$ — C 12 N 5/00, A 61 K 39/395

## II. FIELDS SEARCHED

### Minimum Documentation Searched [7]

| Classification System | Classification Symbols |
|---|---|
| $IPC^4$ | C 12 N 5/00, A 61 K 39/395 |

Documentation Searched other than Minimum Documentation
to the Extent that such Documents are Included in the Fields Searched [8]

## III. DOCUMENTS CONSIDERED TO BE RELEVANT [9]

| Category [*] | Citation of Document, [11] with Indication, where appropriate, of the relevant passages [12] | Relevant to Claim No. [13] |
|---|---|---|
| A | DIABETES, T. 35, March 1986, (US), S.Srikanta et al. "Islet Cell Proteins Defined by Monoclonal Islet Cell Antibody HISL-19", see pages 300-305 -- | 1 |
| A | Scandinavian Journal Of Immunology, 23,1986 (NO), H.Vissing et al. "Monoclonal Antibodies against Pancreatic Islet-Cell-Surface Antigenes Selected by Flow Cytofluorometry", see pages 425-433 ---------- | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 18 April 1989 (18.04.89) | 05 May 1989 (05.05.89) |

| International Searching Authority | Signature of Authorized Officer |
|---|---|
| ISA/SU | |